# EUROPEAN PATENT SPECIFICATION

(11) **EP 3 597 232 B1**
(45) Date of publication and mention of the grant of the patent: **10.01.2024**
(21) Application number: 18767662.2
(22) Date of filing: 14.03.2018
(51) Int. Cl.: B29C 65/20, B29C 65/78, A61M 1/28, A61M 39/14, A61M 39/18, B29K 27/06

(54) **TUBE JOINING DEVICE**
ROHRVERBINDUNGSVORRICHTUNG
DISPOSITIF D'ASSEMBLAGE DE TUBES

(30) Priority: 15.03.2017 JP 2017050351
(43) Date of publication of application: 22.01.2020
(73) Proprietor: Terumo Kabushiki Kaisha, Tokyo 151-0072 (JP)
(72) Inventor: DEMIZU, Syun, Ashigarakami-gun Kanagawa 259-0151 (JP); KOKUBUN, Tomotaka, Ashigarakami-gun Kanagawa 259-0151 (JP)
(74) Representative: TBK
(86) International application number: PCT/JP2018/009940
(87) International publication number: WO 2018/168918

(56) References cited:
- WO-A1-2010/118546
- JP-A- 2013 146 354
- JP-A- 2016 010 480
- JP-A- 2016 010 480

## Description

### Technical Field

The present invention relates to a tube joining device that is used in joining of a tube.

### Background Art

As a technology of connecting tubes formed from a resin to each other, there has been known a joining method in which ends of the tubes formed from a resin are fused and the fused ends are pressed for pressure-joining. The technology has been widely used in various industrial fields, and as an example thereof, an application to a medical technology such as a peritoneal dialysis method has been attempted.

The peritoneal dialysis method is a method in which a predetermined dialysis fluid is put into a body by using a tube (catheter) that is inserted into the abdominal cavity of a patient, and water or waste matters which are transferred to the dialysis fluid through the peritoneum are removed to the outside of the body. When putting the dialysis fluid into the body, the tube that is inserted into the patient is liquid-tightly joined to a tube of a bag in which the dialysis fluid is accommodated. In addition, even when discharging the dialysis fluid from the inside of the body, the tube inserted into the patient is liquid-tightly joined to a liquid discharge bag.

As described above, one tube that becomes a joining target is inserted into the abdominal cavity of the patient. Accordingly, during joining work, it is necessary to pay the closest attention to the work in order for each tube not to be contaminated. In consideration of such circumstances, for example, as described in document JP 2013-146 354 A, atubejoining device capable of automatically performing joining in an aseptic condition by fusing two tubes formed from a resin is developed. In the device, fused ends of the two tubes are replaced and joined, and thus there is no concern of bacterium contamination during joining, and it is possible to maintain sterilization of the tube, the dialysis fluid in the bag, and the like. In addition, in the device, the two tubes are superimposed in an upper and lower direction (height direction) of the device and are set to a close contact state, and a plate-shaped metal wafer that is heated is moved to approach the tubes to perform fusing.

Document JP 2016 - 010 480 A, which is considered closest prior art, discloses a tube connector. Here, a second tube is held in a cover portion of a housing. When closing the cover the second tube comes to lie over a first tube held in the housing.

### Summary of Invention

### Technical Problem

In the case of using the above-described tube joining device, a user such as a patient manually superimposes and sets respective tubes which become a joining target in the device. However, at this time, the tubes have flexibility, and thus the user may fail in handling of the tubes, and may superimpose the tubes in a distorted state or may superimpose the tubes in a three-folded state. When performing fusing-joining work by the device in a state in which the tube is set as described above, the tubes are joined, but a joining failure such as hole occurs at a joining portion.

The invention has been made in consideration of such circumstances, and the object thereof is to provide a tube joining device that allows a user to easily and appropriately perform setting of tubes which become a joining target, and is capable of preventing occurrence of a joining failure caused by a setting error in advance.

### Solution to Problem

According to the invention, there is provided a tube joining device that fuses an end of a first tube and an end of a second tube by a plate-shaped cutting member that is heated, and replaces the fused end of the first tube and the fused end of the second tube and joins the fused ends in an aseptic condition. The tube joining device includes : a housing that includes a cover section that is provided in an openable and closable manner; a first tube holding portion capable of holding any one tube between the first tube and the second tube; a second tube holding portion capable of holding the other tube between the first tube and the second tube; and a tube superimposing portion that moves the other tube to a position at which the other tube is superimposed on the one tube in synchronization with an operation of closing the cover section.

### Advantageous Effects of Invention

According to the tube joining device of the invention, a user sets respective tubes individually in first and second tube holding portions, and performs an operation of causing the tubes to relatively approach each other by covering a cover section. Accordingly, it is possible to easily and appropriately dispose the tubes in a superimposed state. It is not necessary to manually perform work of superimposing the tubes. Accordingly, it is possible to prevent a work error such as setting of the tubes in a distorted state from occurring, and it is possible to prevent a joining failure caused by a tube setting error from occurring in advance. In addition, it is possible to simultaneously perform superimposing of the tubes and preparation work of performing fusing work of the tubes as a previous step by simple work of closing the cover section, and convenience of a user is improved.

### Brief Description of Drawings

Fig. 1 is a perspective view illustrating a tube joining device according to an embodiment of the invention.
Fig. 2 (A) is a view illustrating a configuration example of an operation panel unit that is provided on a front surface portion side of a housing illustrated in Fig. 1, and Fig. 2 (B) is a view illustrating a configuration example of a display unit that is provided on a top surface portion of the housing illustrated in Fig. 1.
Fig. 3 is a perspective view illustrating a schematic arrangement example of constituent elements which are disposed in the housing of the tube joining device.
Fig. 4 is a view illustrating an electric block of a control system of the tube joining device.
Fig. 5(A) is a perspective view illustrating a bottom surface portion of a wafer cassette, and Fig. 5(B) is a perspective view illustrating a top surface portion of the wafer cassette.
Fig. 6 is a perspective view illustrating main portions of the tube joining device in a state in which a cover section is opened.
Fig. 7 is a perspective view illustrating main portions of the tube joining device in a state in which the cover section is closed.
Fig. 8 is a side view illustrating main portions of the tube joining device in a state in which the cover section is opened.
Fig. 9 is a side view illustrating main portions of the tube joining device to explain the course of closing the cover section.
Fig. 10 is a side view illustrating main portions of the tube joining device to explain the course of closing the cover section.
Fig. 11 is a side view illustrating main portions of the tube joining device in a state in which the cover section is closed.
Fig. 12 is a view schematically illustrating tubes which are joined by the tube joining device according to this embodiment.
Fig. 13(A) to Fig. 13(D) are views schematically illustrating respective processes of fusing-joining work by the tube joining device.
Fig. 14 is a view illustrating tubes after joining, in which Fig. 14 (A) is an enlarged view of the tubes after joining, and Fig. 14(B) is a view schematically illustrating an installation state of the tubes after joining.

### Description of Embodiments

Hereinafter, an embodiment of the invention will be described with reference to the accompanying drawings. Furthermore, dimension ratios in the drawings are exaggerated for convenience of explanation, and may be different from actual ratios.

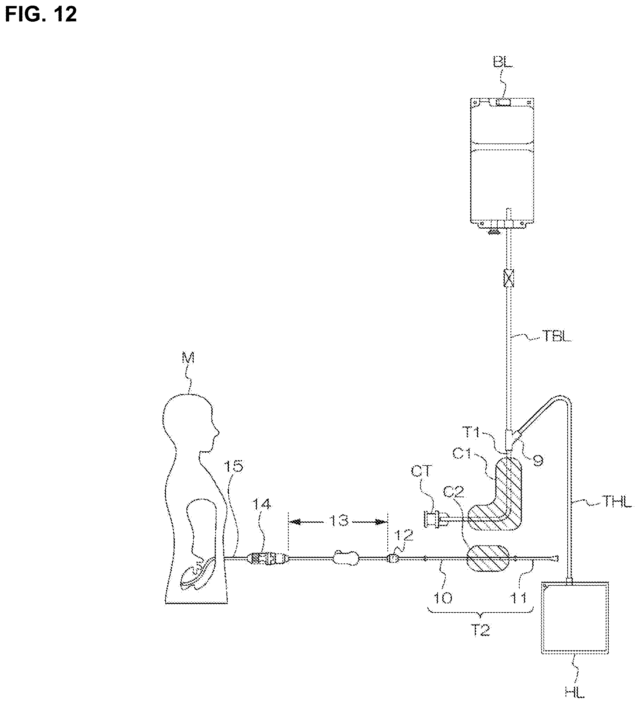

A tube joining device 1 fuses ends of a plurality of tubes T1 and T2 (hereinafter, referred to as a first tube T1 and a second tube T2), and presses and joins the fused ends in an aseptic condition. In this embodiment, description will be given of the tube joining device with reference to an example that is applied to a medical device that is used in joining of a dialysis fluid tube (the first tube T1, corresponds to one tube) of a peritoneal dialysis fluid bag, and a patient peritoneal catheter side tube (the second tube T2, corresponds to the other tube) that is used when performing peritoneal dialysis (refer to Fig. 12) .

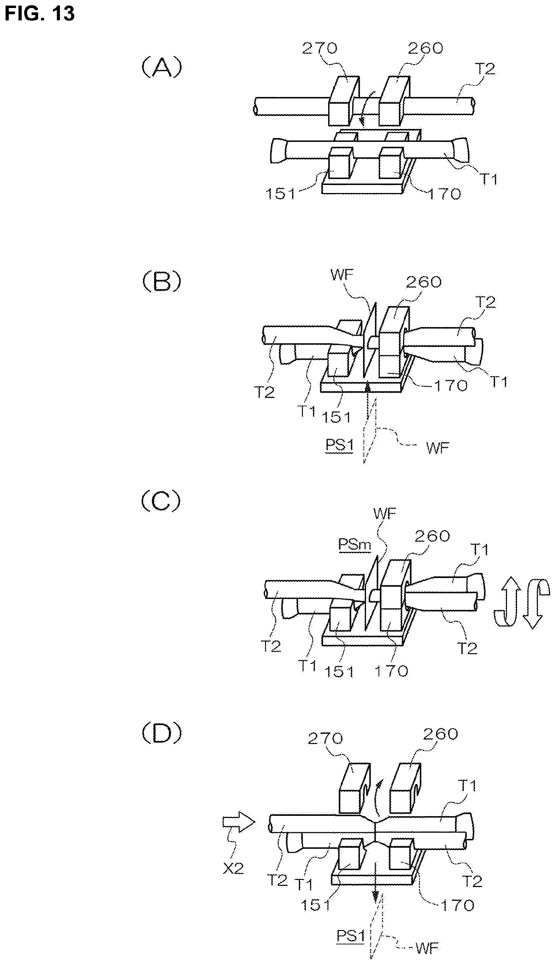

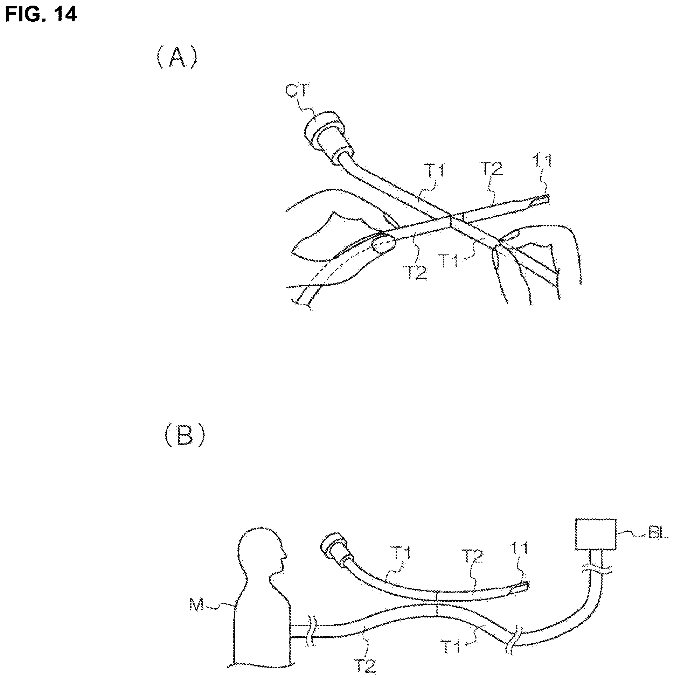

As illustrated in Fig. 13 and Fig. 14, the tube joining device 1 has a configuration in which an end of the first tube T1 and an end of the second tube T2 are fused by a heated wafer WF (correspondingtoaplate-shapedcuttingmember), and replaces and joins the fused end of the first tube T1 and the fused end of the second tube T2.

Respective configurations of the tube joining device 1 will be described.

For example, a preferred use environment of the tube joining device 1 is an environmental temperature of 10°C to 40°C and a relative humidity of 30% to 85%. However, the use environment is not particularly limited as long as the ends of the tubes T1 and T2 can be pressure-welded.

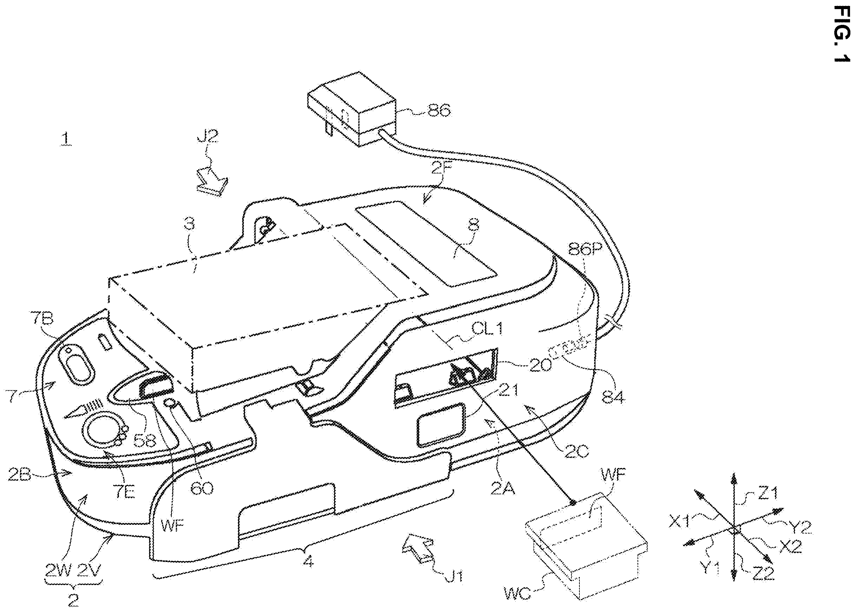

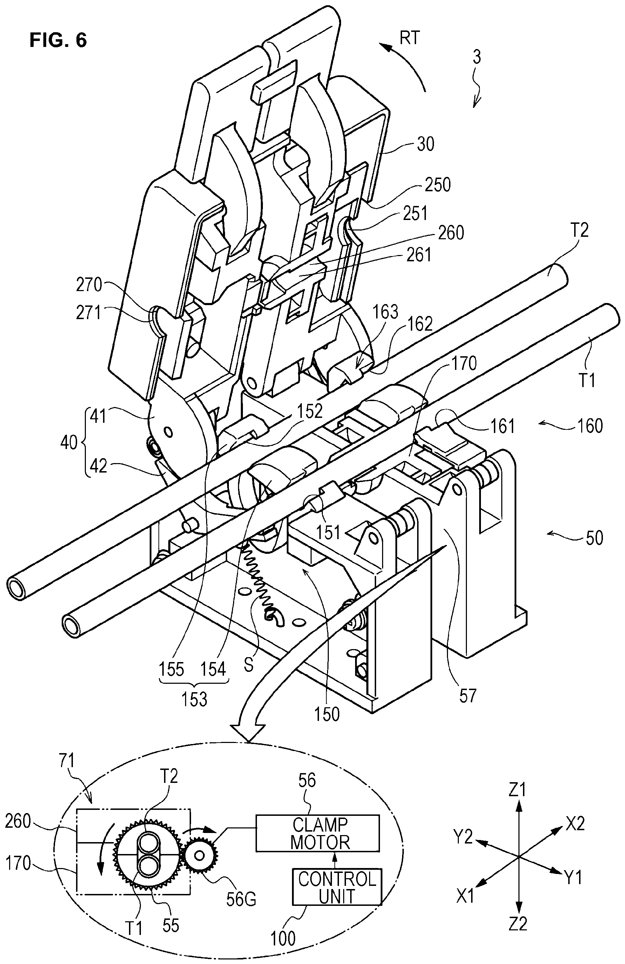

As illustrated in Fig. 1, for example, the tube joining device 1 includes a housing 2 including a cover section 3 that is provided in an openable and closable manner . The cover section 3 rotates around a central axis CL1 in an arrow RS direction to enter an opened state illustrated in Fig. 6 from a closed state illustrated in Fig. 1 after rotating.

For example, the housing 2 has a size of 135 mm (width) × 99 mm (height) × 268 mm (depth), and a weight of approximately 2.4 kg.

The housing 2 includes an upper side housing portion 2W, and a lower side housing portion 2V that is combined to the upper side housing portion 2W.

A tube setting assisting tool 4 is detachably attached to the housing 2. The housing 2 and the tube setting assisting tool 4 are formed from, for example, a hard plastic, but there is no particular limitation to a material, and the like.

As described later, the housing 2 accommodates respective constituent elements of the tube joining device 1. The cover section 3 is disposed on an upper portion of the housing 2.

For example, the housing 2 can be set to a bright color with a relatively high luminosity, specifically, a cream color or a white color. In addition, for example, the tube setting assisting tool 4 may be set to an orange color so that a user (a person who actually uses the tube joining device, a patient, or the like) can visually clearly distinguish the housing 2 and the tube setting assisting tool 4. However, colors of the respective units are not particularly limited, and may be arbitrarily selected.

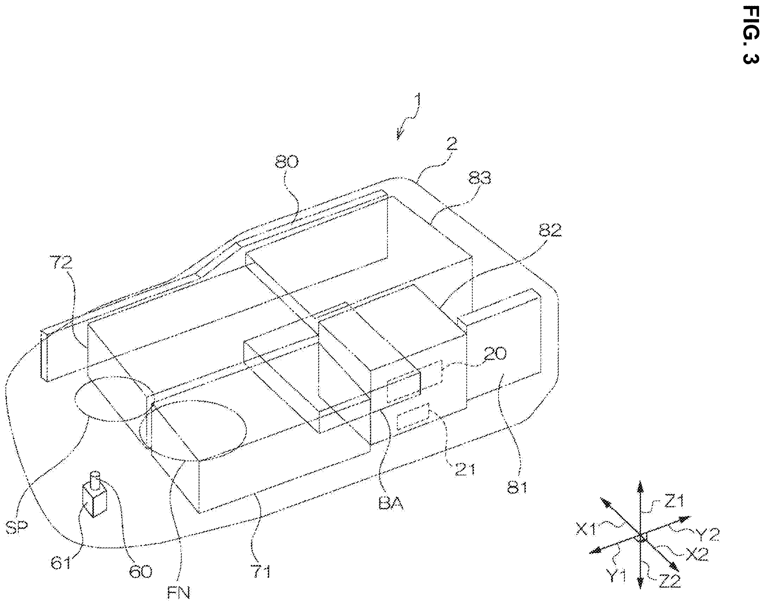

A speaker SP that emits sound, and a fan FN that discharges a gas inside the housing 2 are disposed on a bottom surface portion 2A of the housing 2 (refer to Fig. 3). The fan FN also has a function as a cooling fan that cools down the wafer WF after terminating a joining operation. In the bottom surface portion 2Aof the housing 2, a voice opening that outputs a voice guidance, an alarm sound, and the like which are emitted from the speaker SP to the outside of the housing 2 may be provided, or an exhaust opening for compulsorily emitting heat generated inside the housing 2 or a gas that passes through the inside of the housing 2 to the outside of the housing 2 when the cooling fan FN is operated may be provided.

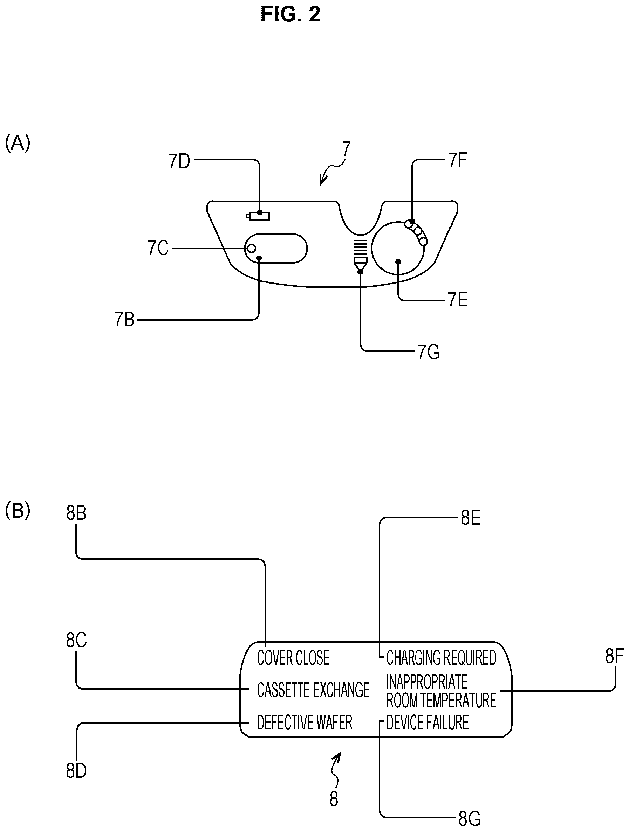

Next, description will be given of an operation panel unit 7 and a display unit 8 with reference to Fig. 2.

Fig. 2 (A) illustrates the operation panel unit 7 that is provided on a front surface 2B side of the housing 2 when viewed from an arrow J1 direction in Fig. 1. Fig. 2(B) illustrates the display unit 8 that is provided on an upper surface portion 2F of the housing 2 illustrated in Fig. 1.

The operation panel unit 7 illustrated in Fig. 2(A) includes a [power] switch button 7B, [power] lamp 7C, a [in-charging] lamp 7D, a [joining] button 7E, a [joining] lamp 7F, and a [wafer taking-out] lamp 7G.

The [power] lamp 7C, the [in-charging] lamp 7D, the [joining] lamp 7F, and the [wafer taking-out] lamp 7G are display lamps indicating various states in the operation panel unit 7. For example, the respective lamps can be constituted by a green light-emitting diode (LED) lamp.

The [power] switch button 7B is a button that is pressed to supply power to the tube joining device 1. The [power] lamp 7C is lightened when pressing the [power] switch button 7B.

The [joining] button 7E is a button that is pressed when a user initiates fusing-joining work of fusing ends of the tubes T1 and T2 and replacing and pressure-joining the ends of the tubes T1 and T2. The [joining] lamp 7F is lightened when the [joining] button 7E is pressed. In addition, the [joining] lamp 7F may be configured to be flickered to give an alarm of a failure state to a user at the time of failure of the tube joining device 1.

The [in-charging] lamp 7D is lightened in a case where charging with respect to a battery BA illustrated in Fig. 3 from a commercial AC power side is performed.

The [wafer taking-out] lamp 7G is lightened or flickered when joining between the tubes T1 and T2 is terminated, and it enters a state in which a user can take out and discharge the wafer WF that has been used from the housing 2.

The display unit 8 illustrated in Fig. 2(B) includes a [cover close] lamp 8B, a [wafer cassette exchange] lamp 8C, a [defective wafer] lamp 8D, a [charging required] lamp 8E, an [inappropriate room temperature] lamp 8F, and a [device failure] lamp 8G.

The [device failure] lamp 8G is an alarm lamp that gives a notification of failure of the tube joining device 1. For example, the [device failure] lamp 8G can be constituted by a red LED lamp. The other lamps are constituted as an alarm display lamp, and can be constituted by, for example, a yellow LED lamp.

When referring to Fig. 1 again, a wafer cassette insertion portion 20 and a wafer cassette taking-out button 21 are provided on a lateral surface portion 2C of the housing 2.

The wafer cassette insertion portion 20 is constituted by a rectangular opening for inserting a wafer cassette WC illustrated in Fig. 1 in a detachable manner. In a state in which the wafer cassette WC is inserted into the housing 2 through the wafer cassette insertion portion 20, when a user pushes the wafer cassette taking-out button 21 with a finger, the wafer cassette WC can be taken out to the outside of the housing 2 through the wafer cassette insertion portion 20. The wafer cassette WC is constituted by a container which stores a plurality of wafers WF which are used in fusing of the tubes T1 and T2.

Next, the tubes T1 and T2 which become a joining target will be described.

Fig. 12 illustrates two tubes T1 and T2 which are joined by the tube joining device 1. As the tube T1 and T2, for example, a vinyl chloride tube can be selected. However, the material of the tubes T1 and T2 is not limited as long as the tubes T1 and T2 can be joined to each other through fusing and pressing. For example, materials of the tubes T1 and T2 may be different from each other.

As illustrated in Fig. 12, a predetermined connector CT is attached to a tip end of the first tube T1. The first tube T1 is connected to a dialysis fluid tube TBL of a dialysis fluid bag BL through a diverging tube 9. In addition, the first tube T1 is connected to a fluid discharge tube THL of a fluid discharge bag HL through the diverging tube 9.

The tube T2 includes an extension tube 10 and a protection tube 11. The extension tube 10 is connected to a peritoneal catheter 15 through a connection tube 12, a silicone tube 13, and a catheter joint 14. One end side of the peritoneal catheter 15 is inserted into an abdominal cavity of a patient M.

The tube joining device 1 fuses a joining portion C1 of the first tube T1 and a joining portion C2 of the second tube T2 by using a heated wafer WF in a state in which the first tube T1 and the second tube T2 are superimposed on each other (refer to Figs. 13(A) and 13(B)). In addition, after the fusing, a fused end of the first tube T1 and a fused end of the second tube T2 are replaced, and the ends are pressed and joined (refer to Figs. 13(C) and 13(D)).

In addition, as illustrated in Fig. 6, before carrying out the joining work by the joining device 1, the first tube T1 and the second tube T2 are held in a housing-side clamp unit 50 provided in the housing 2 so as not to cause a positional deviation. In a state in which the tubes T1 and T2 are held in the joining device 1, through work of closing the cover section 3 (work of causing the cover section 3 to relatively approach the housing 2), the tubes T1 and T2 are set in a superimposing state in which the second tube T2 is set to an upper side and the first tube T1 is set to a lower side (refer to Figs. 13(A) and 13(B)). After the setting, the tubes T1 and T2 are fused by using a predetermined heated wafer WF. Details of the structure of holding the tubes T1 and T2 will be described later.

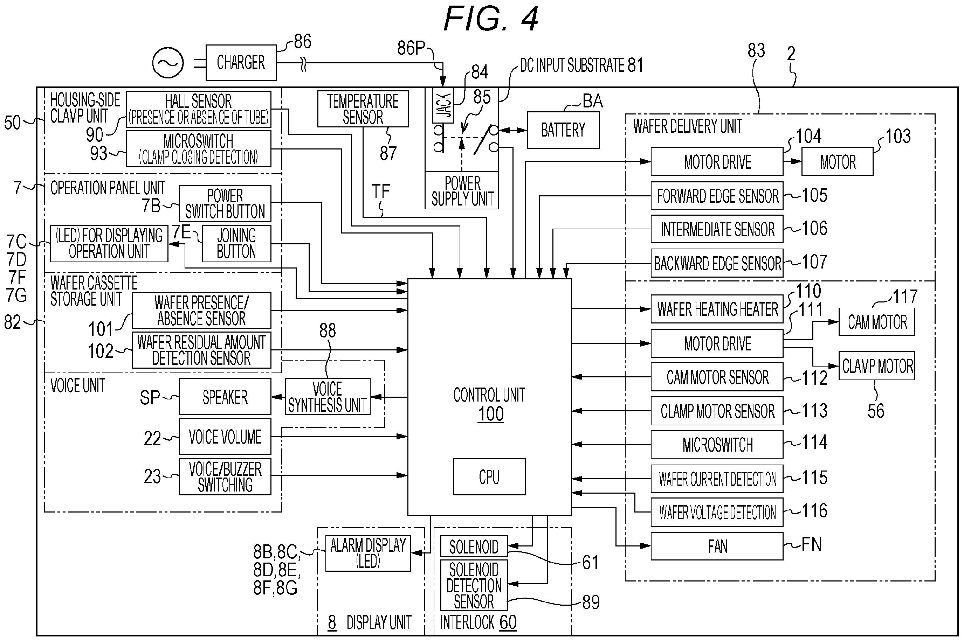

As illustrated in Fig. 1, a take-out port 58 through which the wafer WF that has been used in the fusing is delivered is provided in the vicinity of the operation panel unit 7 of the housing 2. The take-out port 58 is disposed on an extended line of a predetermined gap 57 (refer to Fig. 6) through which the wafer WF passes. Since the take-out port 58 is provided on the extended line of the gap 57, a user can easily take out the wafer WF that is guided to the take-out port 58 by pinching the wafer WF with fingers. An interlock 60 that maintains a closed state of the cover section 3 and a detection sensor 89 which detects an opened or closed state of the cover section 3 during fusing and joining the tubes T1 and T2 are provided in the vicinity of the take-out port 58 (refer to Fig. 3 and Fig. 4).

Fig. 3 is a perspective view illustrating a schematic arrangement example of constituent elements which are disposed in the housing 2 of the tube joining device 1.

As illustrated in Fig. 3, a main substrate 80, a DC input substrate 81, a wafer cassette storage unit 82, a wafer delivery unit 83, a movable clamp unit 71, a solenoid 61, the speaker SP, the fan FN, and a battery BA are accommodated in the housing 2.

For example, it is preferable that the DC input substrate 81 is disposed at a position spaced away from the main substrate 80 as much as possible. The reason for this is to prevent noise from the DC input substrate 81 from having an effect on circuit elements mounted on the main substrate 80.

Next, a function of a control unit 100 of the tube joining device 1 will be described with reference to Fig. 4. Fig. 4 illustrates an electric block of the tube joining device 1.

The tube joining device 1 includes a control unit 100 that collectively controls operations of respective units of the device. The control unit 100 includes a CPU such as a microcomputer, a ROM that stores a control program of the entirety of the device which is executed by the CPU or various pieces of data, and a RAM that temporarily stores measurement data or various pieces of data as a work area.

The control unit 100 is supplied with power from the battery BA on the DC input substrate 81 side. The DC input substrate 81 includes a jack 84 and a switching switch 85. When being connected to a connection pin 86P of a charger 86, the jack 84 receives a predetermined DC power that is AC/DC converted from a commercial AC power supply. Furthermore, the charger 86 and the jack 84 are also illustrated in Fig. 1.

The switching switch 85 connects the jack 84 and the battery BA. DC power from the charger 86 can be used in charging of the battery BA. In addition, the DC power charged in the battery BA is supplied to the control unit 100.

A temperature sensor 87 such as a thermistor is electrically connected to the control unit 100. The temperature sensor 87 detects an environment temperature (outside air temperature) around the housing 2, and supplies outside air temperature information TF to the control unit 100. When heating the tubes T1 and T2, the control unit 100 refers to the outside air temperature information TF, and for example, in a case where the outside air temperature is lower than a temperature that is determined in advance, the control unit 100 executes processing of lengthening a heating time of the tubes T1 and T2. In addition, for example, the control unit 100 performs operation control so that a user is notified of the environment temperature with the speaker SP.

As illustrated in Fig. 4, the [power switch] button 7B, the [joining] button 7E, and the lamps 7C, 7D, 7F, and 7G of the operation panel unit 7 which are illustrated in Fig. 2(A) are electrically connected to the control unit 100.

The speaker SP is electrically connected to the control unit 100 through a voice synthesis unit 88. The speaker SP emits, for example, a voice guidance that is determined in advance in accordance with a command of the control unit 100.

A voice adjusting volume 22 and a voice/message switching switch 23 are electrically connected to the control unit 100. In a case where the voice/message switching switch 23 is "turned on", a voice guidance can be emitted from the speaker SP, and in a case where the voice/message switching switch 23 is "turned off", it is possible to sound a buzzer (not illustrated).

As illustrated in Fig. 4, the [cover close] button 8B, the [wafer cassette exchange] lamp 8C, the [defective wafer] lamp 8D, the [charging required] lamp 8E, the [inappropriate room temperature] lamp 8F, and the [device failure] lamp 8G of the display unit 8 illustrated in Fig. 2(B) are configured to be lightened or flickered in accordance with a command of the control unit 100.

The control unit 100 controls an operation of the interlock 60 to switch a lock state or an unlock state of the cover section 3.

The wafer cassette storage unit 82 includes a wafer presence/absence sensor 101 and a wafer residual amount detection sensor 102. The wafer presence/absence sensor 101 is a sensor that detects whether or not the wafer WF remains in the wafer cassette WC illustrated in Fig. 1. The wafer residual amount detection sensor 102 is a sensor that detects how many sheets of wafers WF remain in the wafer cassette WC illustrated in Fig. 1, that is, the number of sheets of remaining wafer WF. As the wafer presence/absence sensor 101 and the wafer residual amount detection sensor 102, for example, a known photosensor or the like can be used.

The wafer delivery unit 83 is a unit that linearly moves the wafer WF in the wafer cassette WC to a predetermined stand-by position PS1 (refer to Fig. 13(B)). The wafer delivery unit 83 includes a motor 103, a motor drive 104, a forward edge sensor 105, an intermediate sensor 106, and a backward edge sensor 107. When receiving a command from the control unit 100, the motor drive 104 drives the motor 103, and linearly moves the wafer in the wafer cassette WC to the stand-by position PS1 sheet by sheet.

The control unit 100 is electrically connected to a wafer heating heater 110, a motor drive 111, a cam motor sensor 112, a clamp motor sensor 113, a microswitch 114, a wafer current detection unit 115, a wafer voltage detection unit 116, and the fan FN. When the motor drive 111 receives a command from the control unit 100, the motor drive 111 drives the cam motor 117 or the clamp motor 56 to fuse and join the tubes T1 and T2.

The cam motor 117 performs an operation of vertically moving the wafer WF, and an operation of pressing the two tubes T1 and T2 against each other. The operation of vertically moving the wafer WF by the cam motor 117 is an operation of ascending the wafer WF from the stand-by position PS1 to a fusing position PSmon an upward side of the stand-by position PS1, and a descending the wafer WF from the fusing position PSm to the stand-by position PS1 in a contrast manner (refer to Figs. 13 (C) and 13(D)). In addition, the cam motor 117 performs an operation of pressing the tubes T1 and T2 against each other after fusing the tubes T1 and T2. The pressing operation is an operation of causing the wafer WF to enter a stand-by state by descending the wafer WF from the fusing position PSm to the stand-by position PS1, and of pressing and joining the fused end of the first tube T1 and the fused end of the second tube T2 by pressing each of the fused ends against a fused end of a counterpart tube.

The clamp motor 56 performs rotation of the movable clamp unit 71 by 180° and returning rotation after the rotation by 180°.

The cam motor sensor 112 is constituted by, for example, a photosensor that detects a cam position and the original point. The clamp motor sensor 113 is constituted by, for example, a photosensor that detects the original point during rotation of the movable clamp unit 71.

The wafer heating heater 110 is provided to heat a wafer in accordance with a command from the control unit 100. When supplying power, the wafer current detection unit 115 detects a wafer current value that is supplied to the wafer. In addition, the wafer voltage detection unit 116 detects a wafer voltage value that is supplied to the wafer.

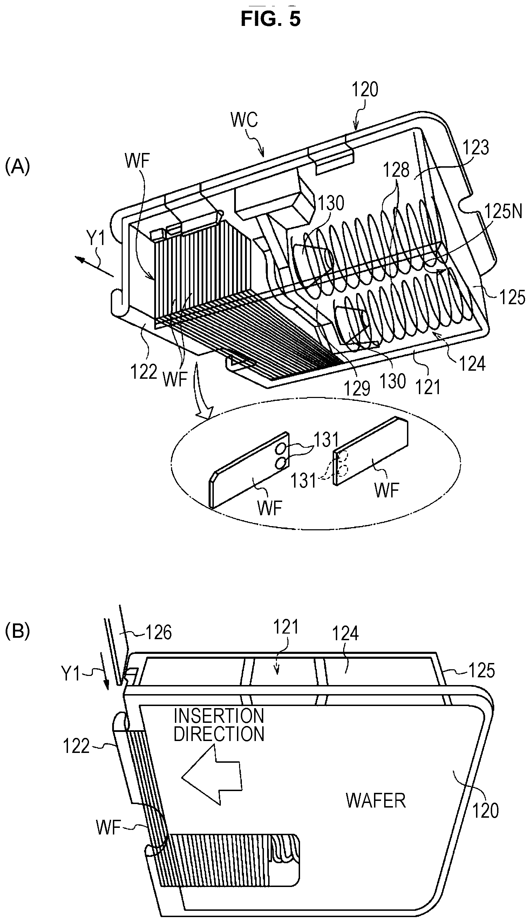

Next, the wafer cassette storage unit 82 and the wafer cassette WC will be described with reference to Fig. 5.

As illustrated in Figs. 5 (A) and 5(B), the wafer cassette WC is constituted by a container for accommodating a plurality of sheets of the wafers WF. The wafer cassette WC is preferably formed from a transparent plastic to visually confirm an inner side of the wafer WF.

The wafer cassette WC includes a top surface portion 120, a bottom surface portion 121, a front surface portion 122, lateral surface portions 123 and 124, and a bottom surface portion 125.

The wafer WF is disposed sheet by sheet on an inner side of the front surface portion 122. In addition, as illustrated in Fig. 5(B), when pressing a pushing member 126 with respect to the wafer WF in a Y1 direction, one sheet of wafer WF is pushed out from the inside of the wafer cassette WC to a predetermined stand-by position PS1 along the Y1 direction.

As illustrated in Figs. 5(A) and 5(B), two springs 128 and a spring accommodation member 129 are accommodated at the inside of the wafer cassette WC. One end of each of the two springs is supported to an inner surface of the bottom surface portion 125 of the wafer cassette WC. On the other hand, the other end of each of the two springs is supported to the spring accommodation member 129. The spring accommodation member 129 includes a positional deviation preventing portion 130 in order for each of the springs 128 not to deviate.

The two springs 128 press a plurality of sheets of the wafers WF against an inner surface of the front surface portion 122 through the spring accommodation member 129. In a state in which the wafers WF are held by the two springs 128, when the pushing member 126 is pressed against the wafer WF located on the front surface portion 122 side in the Y1 direction, only one sheet of the wafer WF located on the outermost side is output from the inside of the wafer cassette WC along the Y1 direction.

As illustrated in Fig. 5(A), the wafer WF that can be used as a cutting member is constituted by a copper metal plate (a thickness: approximately 0.3 mm, a width: approximately 34 mm, and a height: approximately 13 mm) that can be heated by the wafer heating heater 110 (refer to Fig. 4) and is formed in a substantially rectangular shape. Furthermore, the wafer WF has two contact points 131 which are connected to the wafer heating heater 110 when being heated.

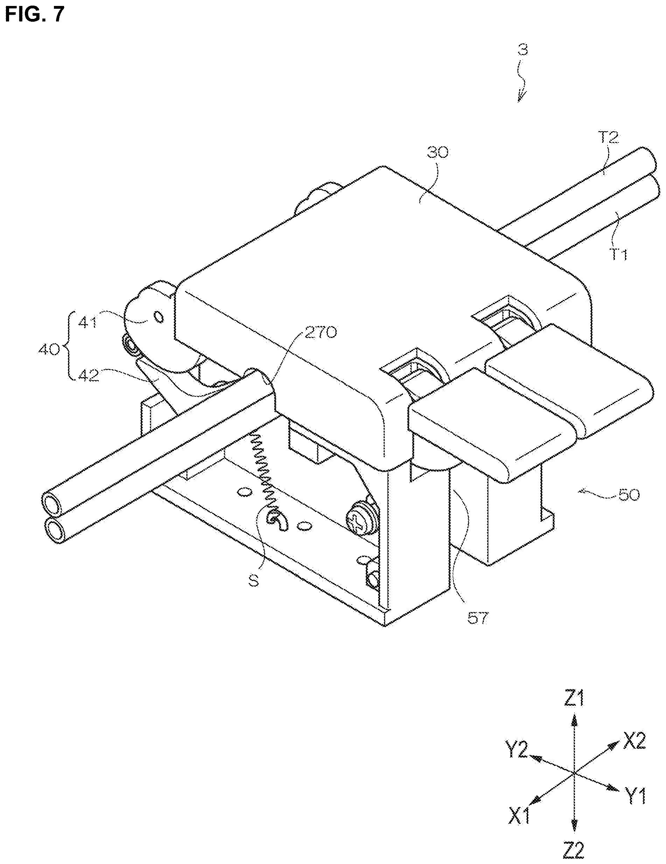

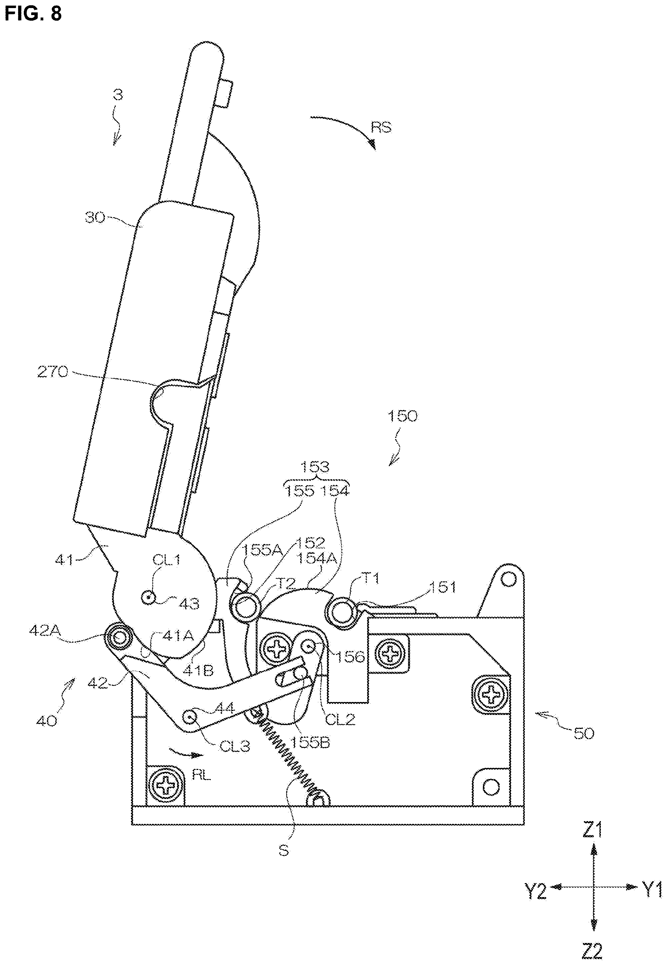

Next, description will be given of a configuration of the housing-side clamp unit 50 that holds and superimposes the tubes T1 and T2 on each other in the tube joining device 1. Fig. 6 and Fig. 7 are perspective views illustrating a state in which the cover section 3 is opened or closed, and Fig. 8 to Fig. 13 are side views when viewed from an arrow J2 direction in Fig. 1.

As illustrated in Fig. 6, the housing-side clamp unit 50 of the housing 2 includes a first accommodation member 150, a secondaccommodationmember160, and a third accommodation member 170. The first accommodation member 150 and the second accommodation member 160 are disposed with a predetermined gap in a disposition direction (extension direction) of the first tube T1. The third accommodation member 170 is disposed between the first accommodation member 150 and the second accommodation member 160.

The first accommodation member 150 includes a first tube holding portion 151 that can hold the first tube T1, a second tube holding portion 152 that can hold the second tube T2, and a tube superimposing portion 153 that moves the second tube T2 to a position at which the second tube T2 is superimposed on the first tube T1 in synchronization with an operation of closing the cover section 3. The second accommodation member 160 includes a first tube holding portion 161 that can hold the first tube T1, a second tube holding portion 162 that can hold the second tube T2, and a tube superimposing portion 163 that moves the second tube T2 to a position at which the second tube T2 is superimposed on the first tube T1 in synchronization with the operation of closing the cover section 3. Since the respective constituent members 151, 152, and 153 of the first accommodation member 150 have the same configurations as those of the respective constituent members 161, 162, and 163 of the second accommodation member 160, in the following description, the respective constituent members 151, 152, and 153 of the first accommodation member 150 will be described in detail, and detailed description of the respective constituent members 161, 162, and 163 of the second accommodation member 160 will be omitted.

For example, the first tube holding portion 151 may be formed by an insertion type groove or the like that is formed in conformity to an outer diameter of the first tube T1, but a structure thereof is not particularly limited as long as the first tube T1 can be fixedly held. The first tube T1 is held in the housing-side clamp unit 50 in a state of being held by the first tube holding portion 151.

As illustrated in Fig. 8, the tube superimposing portion 153 includes a pedestal portion 154 that is disposed in a position-fixed manner, an arm portion 155 that holds the second tube T2 between the arm portion 155 and the pedestal portion 154 and moves the second tube T2 in synchronization with the operation of closing the cover section 3, and a support portion 156 that supports the arm portion 155 to rotate around a central axis CL2 (rotation axis).

The pedestal portion 154 includes a curved surface 154A that is curved in a shape that is convex toward the arm portion 155 side. When the second tube T2 is moved along the curved surface 154A, it is possible to set the second tube T2 in a state of being superimposed on the first tube T1.

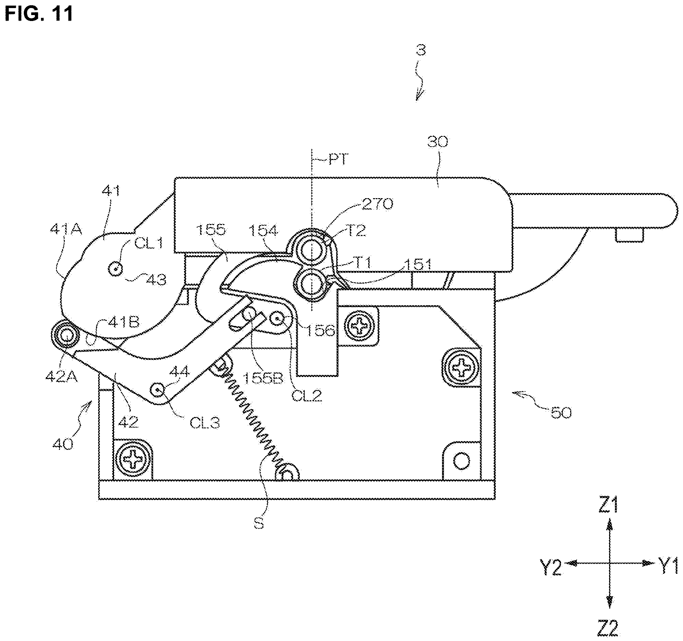

The arm portion 155 guides movement of the second tube T2 along the curved surface 154A (outer surface) of the pedestal portion 154 . The arm portion 155 is configured to release holding of the second tube T2 by a link mechanism 40 as described later before the second tube T2 reaches a superimposing position PT (refer to Fig. 11) at which the second tube T2 is superimposed on the first tube T1.

The arm portion 155 includes a concave groove 155A that holds the second tube T2 between the groove 155A and the curved surface 154A. The arm portion 155 is provided with a protrusion 155B capable of connecting the arm portion 155 and a second link member 42 as described later to each other. An operation of the second link member 42 synchronizes with an operation of the arm portion 155 through the protrusion 155B.

The curved surface 154A of the pedestal portion 154 and the groove 155A of the arm portion 155 function as the "second tube holding portion 152" that movably holds the second tube T2 before the tube superimposing portion 153 superimposes the second tube T2 on the first tube T1 in synchronization with the operation of closing the cover section 3.

The operation of closing the cover section 3, and the operation of moving the second tube T2 by the tube superimposing portion 153 are synchronized by the link mechanism 40. As illustrated in Fig. 8, the link mechanism 40 includes a first link member 41, a second link member 42, a support portion 43 that supports the first link member 41 to rotate around the central axis CL1, and a support portion 44 that supports the second link member 42 to rotate around a central axis CL3.

The first link member 41 opens or closes the cover section 3 by a rotary operation around the central axis CL1. The first link member 41 includes a first curved surface 41A and a second curved surface 41B which are curved in a convex shape.

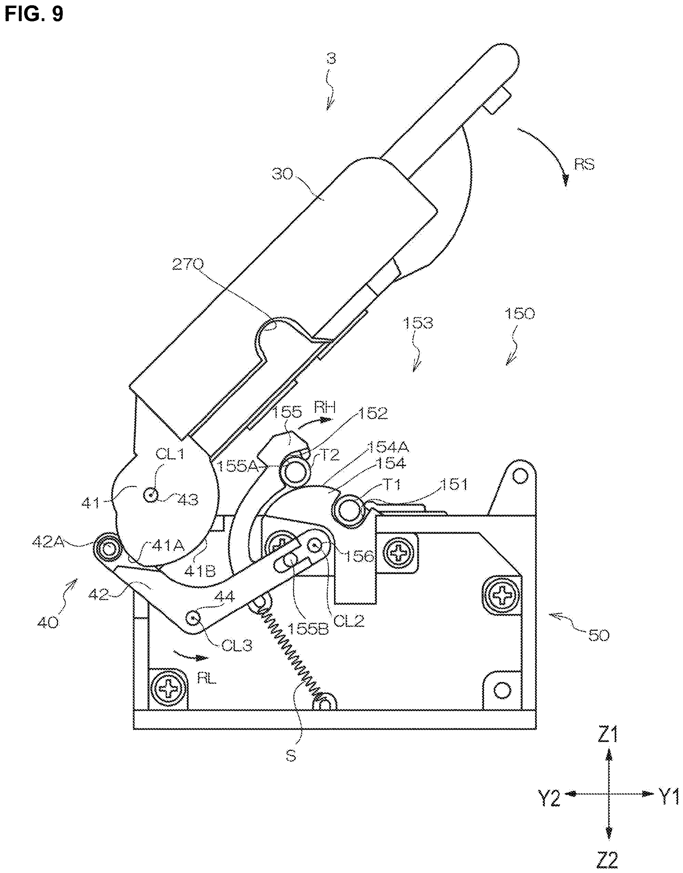

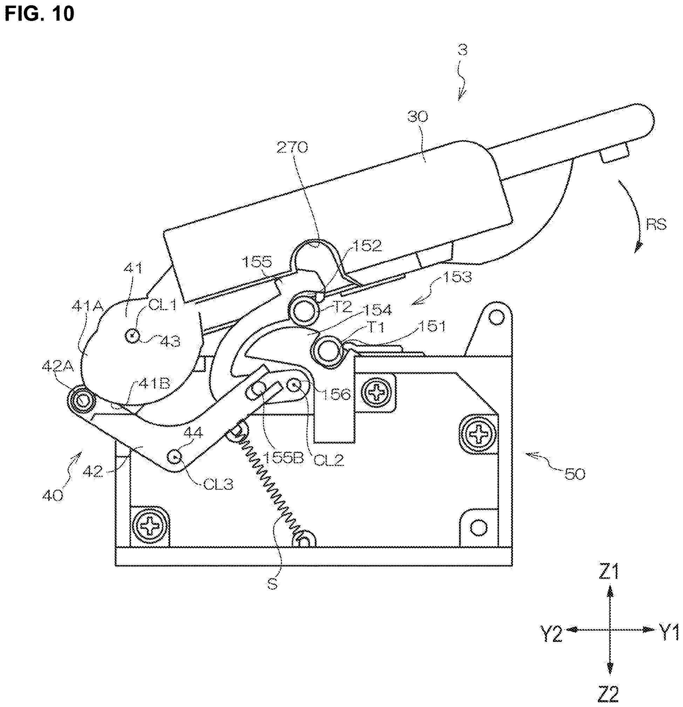

The second link member 42 synchronizes a rotary operation of the first link member 41 around the central axis CL1 and a rotary operation of the arm portion 155 around the central axis CL2 by a rotary operation around the central axis CL3. Specifically, the second link member 42 applies a force to the protrusion 155B attached to the arm portion 155 to rotate the arm portion 155 around the central axis CL2. The second link member 42 is provided with a protrusion 42A that can come into contact with the first curved surface 41A and the second curved surface 41B of the first link member 41. The second link member 42 is pressed due to contact between the protrusion 42A and the first curved surface 41A and rotates around the central axis CL1 in an arrow RL direction (refer to Fig. 9) . In addition, the second link member 42 stops the rotation operation due to contact with the protrusion 42A and the second curved surface 41B, and maintains a rotated state by a predetermined angle (refer to Fig. 10).

In a state in which the cover section 3 is opened, to maintain a state of being disposed at a predetermined position illustrated in Fig. 6, an elastic member S such as a spring that applies a biasing force to the second link member 42 is provided. One end of the elastic member S is fixed to the second link member 42, and the other end is fixed to a bottom surface of the housing 2. According to this, the elastic member S prevents the second link member 42 from carelessly rotating around the central axis CL3 in the arrow RL direction.

At the inside of a clamp plate 30 that is provided in the cover section 3, a structure for holding the tubes T1 and T2 is provided in a similar manner as in the housing-side clamp unit 50. As illustrated in Fig. 6, the clamp plate 30 includes a first accommodation member 250, a second accommodation member 260, and a third accommodation member 270.

The first accommodation member 250 includes a substantially U-shaped groove 251. Similarly, the third accommodation member 270 includes a substantially U-shaped groove 271. The grooves 251 and 271 are provided to hook the second tube T2 when disposing the second tube T2 in the cover section 3.

The second accommodation member 260 includes a concave portion 261 that extends in a disposition direction of the second tube T2. The concave portion 261 is provided to hold the second tube T2.

Furthermore, the shape, the size, and the like of the tube holding portions or the accommodation members which are constructed in the respective portions of the housing-side clamp unit 50 and the cover section 3 are not particularly limited as long as the tubes T1 and T2 can be held or accommodated. In addition, for example, an inclined guide surface or the like may also be formed at the periphery of the tube holding portions or the accommodation members to easily perform setting of the tubes T1 and T2 in the grooves.

Next, a use example of the tube joining device 1 will be described with reference to Fig. 6 to Fig. 11.

For example, as illustrated in Fig. 6, setting of the first tube T1 and the second tube T2 in the housing-side clamp unit 50 can be performed through simple work only by setting the cover section 3 to an opened state, and by pushing the first tube T1 and the second tube T2 from an upward side of the first accommodation member 150 and the second accommodation member 160. Furthermore, the gap 57 that is formed between the first accommodation member 150 and the second accommodation member 160 is a portion to which the wafer WF is guided when fusing the tubes T1 and T2.

As illustrated in Fig. 8, when the cover section 3 is rotated around the central axis CL1 in the arrow RS direction from a state in which the cover section 3 is opened, as illustrated in Fig. 9, the first curved surface 41A of the first link member 41 provided in the cover section 3 comes into contact with the protrusion 42A of the second link member 42.

As illustrated in Fig. 9, in a state in which the first curved surface 41A of the first link member 41 and the protrusion 42A of the second link member 42 come into contact with each other, the cover section 3 is further closed, the protrusion 42A of the second link member 42 is pressed by the first curved surface 41A of the first link member 41 in the arrow RL direction. According to this, the second link member 42 rotates around the central axis CL3 in the arrow RL direction. When the second link member 42 rotates, the arm portion 155 of the tube superimposing portion 153 rotates around the central axis CL2 in an arrow RH direction. According to this, the arm portion 155 can move the second tube T2 in a direction in which the second tube T2 approaches the first tube T1 along the curved surface 154A of the pedestal portion 154. At this time, the arm portion 155 of the tube superimposing portion 153 holds and moves the second tube T2 between the curved surface 154A of the pedestal portion 154 and the groove 155A of the arm portion 155.

When the second link member 42 rotates in the arrow RL direction by a predetermined angle, as illustrated in Fig. 10, the second curved surface 41B of the first link member 41 comes into contact with the protrusion 42A of the second link member 42. According to this, pressing of the first link member 41 against the second link member 42 in the arrow RL direction is released, and rotation of the second link member 42 stops. Accordingly, the armportion 155 rotates in the arrow RH direction by a predetermined angle, and then rotation thereof stops. According to this, holding of the second tube T2 by the tube superimposing portion 153 is released before the second tube T2 reaches the superimposing position PT (refer to Fig. 11) at which the second tube T2 is superimposed on the first tube T1.

After holding of the second tube T2 by the tube superimposing portion 153 is released, the third accommodation member 270 of the clamp plate 30 holds the second tube T2 in substitution for the tube superimposing portion 153. In addition, when the cover section 3 is closed, as illustrated in Fig. 11, in a state in which the second tube T2 is accommodated and held by the third accommodation member 270, the second tube T2 moves to approach the first tube T1. Furthermore, in the case of the tube superimposing portion 163, the first accommodation member 250 and the second accommodation member 260 hold the second tube T2 in substitution for the tube superimposing portion 163.

Then, when the cover section 3 is closed, as illustrated in Fig. 11, in a state in which the second tube T2 is held in the third accommodation member 270, and the first tube T1 is held in the first tube holding portion 151, the first tube T1 and the second tube T2 are set in a superimposed state in which the second tube T2 becomes an upper side and the first tube T1 becomes a lower side. As illustrated in Fig. 7, when the cover section 3 is closed, the inside of the clamp plate 30 and the inside of the housing-side clamp unit 50 are isolated from the outside. Accordingly, it is possible to perform fusing and joining of the tubes T1 and T2 disposed between the clamp plate 30 and the housing-side clamp unit 50 in an aseptic condition.

As illustrated in Fig. 7, when the cover section 3 is closed, the second accommodation member 260 of the clamp plate 30 and the third accommodation member 170 of the housing-side clamp unit 50 are integrated with each other. The integrated second accommodation member 260 and third accommodation member 170 form the movable clamp unit 71 that replaces ends of the tubes T1 and T2 which are fused by the wafer WF.

As is simply illustrated in a broken line portion in Fig. 6, for example, a predetermined gear 55 can be formed at the periphery of the second accommodation member 260 of the clamp plate 30 and the periphery of the third accommodation member 170 of the housing-side clamp unit 50. In addition, the gear 55 can be configured to engage with a gear 56G of the clamp motor 56 that drives an operation of replacing positions of ends of the tubes T1 and T2 after fusing the tubes T1 and T2.

For example, when the clamp motor 56 operates by a command of a control unit 100 (refer to Fig. 4) and rotates the gear 56G, the second accommodation member 260 of the clamp plate 30 and the third accommodation member 170 of the housing-side clamp unit 50 positively rotate by 180° or reversely rotate by 180° in an integrated state. In addition, as illustrated in Fig. 13(C), during the rotation, one end side (the right side in the drawing) of the first tube T1 and the second tube T2 which are fused rotates, and the other end side is pressed by the third accommodation member 270 of the clamp plate 30 and the first tube holding portion 151 of the housing-side clamp unit 50 and is held therebetween. As a result, a position of the end of the first tube T1 after being fused and a position of the end of the second tube T2 after being fused are vertically reversely rotated by 180°. According to this, one end of the second tube T2 is located on an upper side and one end of the first tube T1 is located on a lower side before being fused, but the one end of the first tube T1 is located on an upper side and the one end of the second tube T2 is located on a lower side after being fused.

Next, a work process of joining the tubes T1 and T2 will be described with reference to Fig. 13, Fig. 14, and other drawings. Fig. 13 and Fig. 14 are views schematically illustrating a fusing-joining work flow by the tube joining device 1.

First, as illustrated in Fig. 6, a user sets the first tube T1 and the second tube T2 in the housing-side clamp unit 50. According to this, the first tube T1 is held by the first tube holding portions 151 and 161, and the third accommodation member 170, and the second tube T2 is held by the tube superimposing portions 153 and 163.

Next, as illustrated in Fig. 7, the user moves the cover section 3 to approach the housing-side clamp unit 50, and closes the cover section 3. In addition, through the work, the second tube T2 is superimposed on the first tube T1. As described above, holding of the second tube T2 by the tube superimposing portions 153 and 163 is released before the second tube T2 reaches the superimposing position PT (refer to Fig. 11) at which the second tube T2 is superimposed on the first tube T1, that is, before the cover section 3 is closed. According to this, as illustrated in Fig. 13(A), the first accommodation member 250, the second accommodation member 260, and the third accommodation member 270 hold the second tube T2 in substitution for the tube superimposing portions 153 and 163.

The user pushes the joining button 7E of the operation panel unit 7 after closing the clamp plate 30 (refer to Fig. 2(A)). Then, the fusing work by the wafer WF is initiated.

When the fusing work is initiated, as illustrated in Fig. 13(B), the wafer WF is moved to the stand-by position PS1 that is located on a downward side of the tubes T1 and T2 from the wafer cassette WC. At this time, the wafer WF is heated to, for example, approximately 300°C through heating by the wafer heating heater 110. As illustrated in Fig. 13(B), the heated wafer WF ascends from the stand-by position PS1 indicated by a broken line to the fusing position PSm indicated by a solid line in accordance with an operation of the cam motor 117. As a result, the tubes T1 and T2 are fused by the wafer WF.

Next, as illustrated in Fig. 13(C), the clamp motor 56 rotates the gear 56G (refer to Fig. 6) . The second accommodation member 260 of the clamp plate 30 and the third accommodation member 170 of the housing-side clamp unit 50 rotate by 180° while holding the first tube T1 and the second tube T2. As a result, the fused end of the first tube T1 is disposed on an upper side, and the fused end of the second tube T2 is disposed on a lower side.

Next, as illustrated in Fig. 13(D), the wafer WF descends from the fusing position PSm to the stand-by position PS1, and the tubes T1 and T2 which are located on a non-rotation side are pushed in an X2 direction. According to this, an end of the first tube T1 that is located on one side rotated by 180° is pressed and joined to an end of the second tube T2 on the other side that is not rotated. In addition, an end of the second tube T2 that is located on the one side rotated by 180° is pressed and joined to an end of the first tube T1 that is located on the other side that is not rotated. Then, the tubes T1 and T2 are cooled down, and thus joining is completed.

After the joining is completed, the wafer WF that has been used in the fusing is discharged to the take-out port 58. The user can take out the wafer WF that has been used from the take-out port 58 by pinching the wafer WF with fingers.

Then, the user lifts up the cover section 3 in the arrow RT direction illustrated in Fig. 6. In this manner, the cover section 3 is detached from the housing-side clamp unit 50. As illustrated in Figs. 14(A) and 14(B), the user detaches and separates the tubes T1 and T2 after joining from the housing-side clamp unit 50. As described above, it is possible to conveniently join the tube T1 on the dialysis fluid bag BL side and the tube T2 on a user M side as illustrated in Fig. 12 in an aseptic condition. Furthermore, it is also possible to construct the tube joining device 1 in such a manner that separation of the tubes T1 and T2 which remain in the housing-side clamp unit 50 after joining is performed, for example, in synchronization with the operation of lifting up the cover section 3.

As described above, the tube joining device 1 according to this embodiment is a tube joining device that fuses an end of the first tube T1 and an end of the second tube T2 by a heated wafer WF (plate-shaped cutting member), and replaces the fused end of the first tube T1 and the fused end of the second tube T2 and joins the fused ends in an aseptic condition. The tube joining device 1 includes the housing 2 including the cover section 3 that is provided in an openable and closable manner, the first tube holding portions 151 and 161 which are capable of holding the first tube T1, the second tube holding portions 152 and 162 which are capable of holding the second tube T2, and the tube superimposing portions 153 and 163 which move the second tube T2 to a position at which the second tube T2 is superimposed on the first tube T1 in synchronization with an operation of closing the cover section 3.

According to the tube joining device 1 configured as described above, a user sets the tubes T1 and T2 individually in the first and second tube holding portions 151, 152, 161, and 162, and performs an operation of causing the tubes T1 and T2 to relatively approach each other by closing the cover section 3. According to this, the user can easily and appropriately dispose the tubes T1 and T2 in a superimposed state. It is not necessary to manually perform work of superimposing the tubes T1 and T2. Accordingly, it is possible to prevent a work error such as setting of the tubes in a distorted state from occurring, and it is possible to prevent a joining failure caused by a tube setting error from occurring in advance. In addition, it is possible to simultaneously perform superimposing of the tubes T1 and T2 and preparation work of performing fusing work of the tubes T1 and T2 as a previous step by simple work of closing the cover section 3, and convenience of a user is improved.

In addition, the tube superimposing portion 153 includes the pedestal portion 154 that is disposed in a position-fixed manner, and the arm portion 155 that holds the second tube T2 between the arm portion 155 and the pedestal portion 154 and moves the second tube T2 in synchronization with the operation of closing the cover section 3. The arm portion 155 guides movement of the second tube T2 along an outer surface of the pedestal portion 154. According to this, it is possible to easily and appropriately dispose the tubes T1 and T2 in a superimposed state through work of closing the cover section 3.

In addition, the arm portion 155 is configured to release the holding of the second tube T2 before the second tube T2 reaches the superimposing position PT at which the second tube T2 is superimposed on the first tube T1. Accordingly, in the fusing work and the joining work of the tubes T1 and T2, the arm portion 155 does not interfere movement of the second tube T2, and thus it is possible to smoothly perform the fusing work and the joining work.

In addition, the pedestal portion 154 includes the curved surface 154A that is curved in a shape that is convex toward the arm portion 155 side, and the arm portion 155 includes the concave groove 155A that holds the second tube T2 between the arm portion 155 and the curved surface 154A. Accordingly, it is possible to more reliably hold the second tube T2 to dispose the tubes T1 and T2 in a superimposed state.

In addition, the first tube holding portion 151, the second tube holding portion 152, and the tube superimposing portion 153 are provided in the housing 2, and the tube superimposing portion 153 functions as the second tube holding portion 152 that holds the second tube T2 in a movable manner before the second tube T2 is superimposed on the first tube T1. Accordingly, it is possible to decrease a movement distance of the second tube T2 to the minimum, and it is possible to easily dispose the tubes T1 and T2 in a superimposed state within a short time.

Hereinbefore, description has been given of the tube joining device according to the invention with reference to the embodiment, but the invention is not limited to the configuration described in the embodiment, and can be appropriately modified on the basis of the appended claims.

For example, the configuration of the housing or the respective portions of the tube joining device can be modified in correspondence with a use and a purpose of the device, design circumstances and the like, and thus there is no limitation to the configuration illustrated in the drawing. For example, the work of superimposing the first tube and the second tube may be configured to be performed by relative approaching movement of the first tube holding portion and the second tube holding portion which respectively hold the tubes. For example, the configuration of the work may be changed so that the tubes are superimposed on each other by causing the first tube holding portion side to perform approaching movement without causing the second tube holding portion side to perform approaching movement.

In addition, the tubes which become a joining target may be tubes of which positions of ends after being fused are replaced with each other and are subjected to pressure-joining, and there is no limitation to the tubes used in the peritoneal dialysis.

In addition, description has been given of an example in which the first tube holding portion holds the first tube, and the second tube holding portion holds the second tube, but there is no limitation thereto. The first tube holding portion can hold the second tube, and the second tube holding portion can hold the first tube.

### Reference Signs List

- 1: TUBE JOINING DEVICE
- 2: HOUSING
- 3: COVER SECTION
- 30: CLAMP PLATE
- 50: HOUSING-SIDE CLAMP UNIT
- 100: CONTROL UNIT
- 151, 161: FIRST TUBE HOLDING PORTION
- 152, 162: SECOND TUBE HOLDING PORTION
- 153, 163: TUBE SUPERIMPOSING PORTION
- 154: PEDESTAL PORTION
- 154A: CURVED SURFACE
- 155: ARM PORTION
- 155A: GROOVE
- T1: FIRST TUBE (ONE TUBE)
- T2: SECOND TUBE (THE OTHER TUBE)
- WF: WAFER (CUTTING MEMBER)
- PT: SUPERIMPOSING POSITION

## Claims

1. A tube joining device (1) that fuses an end of a first tube (T1) and an end of a second tube (T2) by a plate-shaped cutting member (WF) that is heated, and replaces the fused end of the first tube (T1) and the fused end of the second tube (T2) and joins the fused ends in an aseptic condition, the tube joining device (1) comprising:
a housing (2) that includes a cover section (3) that is provided in an openable and closable manner;
a first tube holding portion (151, 161) capable of holding any one tube (T1) out of the first tube (T1) and the second tube (T2);
a second tube holding portion (152, 162) capable of holding the other tube (T2) out of the first tube (T1) and the second tube (T2); and
a tube superimposing portion (153, 163);
**characterized in that**
the tube superimposing portion (153, 163) is configured to move the other tube (T2) to a position at which the other tube (T2) is superimposed on the one tube (T1) in synchronization with an operation of closing the cover section (3).

2. The tube joining device (1) according to claim 1,
wherein the tube superimposing portion (153, 163) includes,
a pedestal portion (154) that is disposed in a position-fixed manner, and
an arm portion (155) that holds the other tube between the arm portion (155) and the pedestal portion (154), and moves the other tube in synchronization with the operation of closing the cover section (3), and
the arm portion (155) guides movement of the other tube along an outer surface of the pedestal portion (154).

3. The tube joining device (1) according to claim 2, wherein the arm portion (155) releases holding of the other tube before the other tube reaches a superimposing position at which the other tube is superimposed on the one tube.

4. The tube joining device (1) according to claim 2 or 3,
wherein the pedestal portion (154) includes a curved surface that is curved in a shape that is convex toward the arm portion (155) side, and
the arm portion (155) includes a concave groove that holds the other tube between the arm portion (155) and the curved surface.

5. The tube joining device (1) according to any one of claims 1 to 4,
wherein the first tube holding portion (151, 161), the second tube holding portion (152, 162), and the tube superimposing portion (153, 163) are provided in the housing (2), and
the tube superimposing portion (153, 163) functions as the second tube holding portion (152, 162) that holds the other tube in a movable manner before the other tube is superimposed on the one tube.

## Patentansprüche

1. Schlauchverbindungsvorrichtung (1), die ein Ende eines ersten Schlauchs (T1) und ein Ende eines zweiten Schlauchs (T2) durch ein plattenförmiges Schneidelement (WF), das erwärmt wird, miteinander verschmilzt und das verschmolzene Ende des ersten Schlauchs (T1) und das verschmolzene Ende des zweiten Schlauchs (T2) ersetzt und die verschmolzenen Enden in einem aseptischen Zustand miteinander verbindet, wobei die Schlauchverbindungsvorrichtung (1) umfasst
ein Gehäuse (2), das einen Deckelabschnitt (3) hat, der öffenbar und verschließbar bereitgestellt ist;
einen ersten Schlauchhalteabschnitt (151, 161), der in der Lage ist, einen beliebigen Schlauch (T1) aus dem ersten Schlauch (T1) und dem zweiten Schlauch (T2) zu halten;
einen zweiten Schlauchhalteabschnitt (152, 162), der in der Lage ist, den anderen Schlauch (T2) aus dem ersten Schlauch (T1) und dem zweiten Schlauch (T2) zu halten; und
einen Schlauchüberlagerungsabschnitt (153, 163);
**dadurch gekennzeichnet, dass**
der Schlauchüberlagerungsabschnitt (153, 163) so konfiguriert ist, dass er den anderen Schlauch (T2) zu einer Position bewegt, in der der andere Schlauch (T2) auf dem einen Schlauch (T1) in Synchronisation mit einem Vorgang des Schließens des Deckelabschnitts (3) überlagert ist.

2. Schlauchverbindungsvorrichtung (1) gemäß Anspruch 1,
wobei der Schlauchüberlagerungsabschnitt (153, 163) folgendes aufweist,
einen Sockelabschnitt (154), der in einer positionsfixierten Weise vorgesehen ist, und
einen Armabschnitt (155), der den anderen Schlauch zwischen dem Armabschnitt (155) und dem Sockelabschnitt (154) hält und den anderen Schlauch synchron mit dem Vorgang des Schließens des Deckelabschnitts (3) bewegt, und
der Armabschnitt (155) die Bewegung des anderen Schlauchs entlang einer äußeren Oberfläche des Sockelabschnitts (154) führt.

3. Schlauchverbindungsvorrichtung (1) gemäß Anspruch 2, wobei der Armabschnitt (155) das Halten des anderen Schlauchs aufhebt, bevor der andere Schlauch eine Position erreicht, in der der andere Schlauch auf den einen Schlauch überlagert ist.

4. Schlauchverbindungsvorrichtung (1) gemäß Anspruch 2 oder 3,
wobei der Sockelabschnitt (154) eine gekrümmte Oberfläche hat, die in einer Form gekrümmt ist, die zur Seite des Armabschnitts (155) hin konvex ist, und
der Armabschnitt (155) eine konkave Nut hat, die den anderen Schlauch zwischen dem Armabschnitt (155) und der gekrümmten Fläche hält.

5. Schlauchverbindungsvorrichtung (1) gemäß einem der Ansprüche 1 bis 4,
wobei der erste Schlauchhalteabschnitt (151, 161), der zweite Schlauchhalteabschnitt (152, 162) und der Schlauchüberlagerungsabschnitt (153, 163) in dem Gehäuse (2) bereitgestellt sind, und
der Schlauchüberlagerungsabschnitt (153, 163) als der zweite Schlauchhalteabschnitt (152, 162) fungiert, der den anderen Schlauch beweglich hält, bevor der andere Schlauch über den einen Schlauch überlagert wird.

## Revendications

1. Dispositif d'assemblage de tubes (1) qui fusionne une extrémité d'un premier tube (T1) et une extrémité d'un second tube (T2) par un élément de coupe en forme de plaque (WF) qui est chauffé, et qui remplace l'extrémité fusionnée du premier tube (T1) et l'extrémité fusionnée du second tube (T2) et assemble les extrémités fusionnées dans un état aseptique, le dispositif d'assemblage de tubes (1) comprenant :
un boîtier (2) qui comprend une section de couvercle (3) qui est fournie de manière à pouvoir être ouverte et fermée ;
une première partie de retenu de tube (151, 161) capable de maintenir n'importe quel tube (T1) hors du premier tube (T1) et du second tube (T2) ;
une seconde partie de retenu de tube (152, 162) capable de maintenir l'autre tube (T2) hors du premier tube (T1) et du second tube (T2) ; et
une partie de superposition de tubes (153, 163) ;
**caractérisé en ce que**
la partie de superposition de tube (153, 163) est configurée pour déplacer l'autre tube (T2) vers une position dans laquelle l'autre tube (T2) est superposé sur le premier tube (T1) en synchronisation avec une opération de fermeture de la section de couverture (3).

2. Dispositif d'assemblage de tubes (1) selon la revendication 1,
la partie de superposition de tube (153, 163) comprenant : une partie de piédestal (154) qui est disposée de manière fixe, et
une partie de bras (155) qui retient l'autre tube entre la partie de bras (155) et la partie de piédestal (154), et déplace l'autre tube en synchronisation avec l'opération de fermeture de la section de couvercle (3), et
la partie de bras (155) guidant le mouvement de l'autre tube le long d'une surface extérieure de la partie de piédestal (154).

3. Dispositif d'assemblage de tubes (1) selon la revendication 2, la partie de bras (155) relâchant la retenu de l'autre tube avant que l'autre tube n'atteigne une position de superposition au niveau de laquelle l'autre tube est superposé sur le premier tube.

4. Dispositif d'assemblage de tubes (1) selon la revendication 2 ou 3,
la partie de piédestal (154) comprenant une surface incurvée de forme convexe vers le côté de la partie de bras (155), et
la partie de bras (155) comprenant une rainure concave qui retient l'autre tube entre la partie de bras (155) et la surface incurvée.

5. Dispositif d'assemblage de tubes (1) selon l'une quelconque des revendications 1 à 4,
la première partie de retenu de tube (151, 161), la seconde partie de retenu de tube (152, 162) et la partie de superposition de tube (153, 163) étant fournies dans le boîtier (2), et
la partie de superposition de tube (153, 163) fonctionnant comme la seconde partie de retenu de tube (152, 162) qui retient l'autre tube de manière mobile avant que l'autre tube ne soit superposé sur le premier tube.
